# EUROPEAN PATENT APPLICATION

(11) **EP 1 457 555 A1**
(43) Date of publication of application: **15.09.2004**
(21) Application number: 02786175.6
(22) Date of filing: 20.12.2002
(51) Int. Cl.: C12N 15/09, C12N 5/04, C12Q 1/02, C12Q 1/68

(54) **METHODS FOR IDENTIFYING GIBBERELLIN RESPONSIVE GENES USING CULTURED PLANT CELLS**

(30) Priority: 21.12.2001 JP 2001388993
(71) Applicant: National Institute of Agrobiological Sciences, Tsukuba-shi, Ibaraki 305-8602 (JP)
(72) Inventor: MINAMI, Eiichi, c/o Nat.Inst.Agrobiol.Sciences, Tsukuba-shi, Ibaraki 305-8602 (JP); SHIBUYA, Naoto, Nat. Inst. of Agrobio. Sciences, Tsukuba-shi, Ibaraki305-8602 (JP); DAY, Robert, B., Nat. Inst. of Agrobio. Sciences, Tsukuba-shi, Ibaraki 305-8602 (JP)
(74) Representative: Keller, Günter, Dr.
(86) International application number: PCT/JP2002/013376
(87) International publication number: WO 2003/054192

(57) **Abstract**

To date, those skilled in the art have never considered that cultured plant cells respond to gibberellins. However, to comprehensively and conveniently identify and isolate gibberellin responsive genes, cultured cells, which are uniform and easy to handle, are appropriate. Considering the advantages of cultured cells, the present inventors developed methods for identifying and isolating gibberellin responsive genes using cultured cells. After pre-culturing cells in an auxin-free medium, they examined whether or not changes in gene expression could be detected. As a result, it was surprisingly revealed that changes in the expression of some genes could be detected after gibberellin treatment.

## Description

### Technical Field

The present invention relates to methods for identifying and screening gibberellin responsive genes using cultured plant cells, and methods for using cultured plant cells to determine the presence or absence of gibberellin responsiveness in tested genes.

### Background Art

Gibberellins are a group of plant hormones that play important roles in the life cycle of plants, including roles in germination, elongation, and flower bud initiation. Therefore, gibberellin responsive genes are expected to have widespread applications in the creation of transgenic crops in which the above-mentioned germination, elongation, flower bud initiation, and so on has been modified.

To date, the following have been reported as genes whose expression is induced by gibberellins: starch hydrolyzing enzyme (α-amylase) gene in germinating seeds, barley Gamyb (transcription regulator of α-amylase gene), rice OsCa-atpase (Ca²⁺-ATPase), rice UBC (Ubiquitin-conjugating enzyme), oat Aspk10 (ribosomal protein kinase) , rice Os-Exp2 and 4 (expansin, cell wall protein) , rice cyc0s1,2 (mitotic cyclin), rice cdc2Os-2 (cdc2 homologue), rice R2 (cdc2 activating kinase) , rice RPA1 (replication protein A1), cucumber CHRC (carotenoid associated protein), Arabidopsis GASA1 and GASA4, and cucumber CRG16 (Shinjiro Yamaguchi, "Signal transduction of plant hormones", Plant Cell Technology Series 10, edited by Hiroo Fukuda, Yasunori Machida, Yuji Kamiya, and Tsukaho Hattori (1998) pp. 97-106). Four of the rice expansin genes, OsEXPB3 , OsEXPB4, OsEXPB6 , and OsEXPB11, have also been reported to be induced by gibberellins (Lee, Y., and Kende, H., Plant Physiol. (2001) 127: 645-654). In addition, expression of the following genes has been reported to be suppressed by gibberellins: genes involved in gibberellin biosynthesis, oat Aspk9 (MAP kinase), Vigna AB-ACO1 (ACC oxidase), Arabidopsis GA4 (gibberellin 3β-hydroxylase), and Arabidopsis At2301 (GA5, gibberellin 20-oxidase) (Shinjiro Yamaguchi, "Signal transduction of plant hormones", Plant Cell Technology Series 10, edited by Hiroo Fukuda, Yasunori Machida, Yuji Kamiya, and Tsukaho Hattori (1998) pp. 97-106).

Gibberellin responsiveness has been identified by using whole plants or slices thereof. However, whole plants and slices thereof are composed of heterogeneous cells and are difficult to handle. Therefore, comprehensive, convenient identification and isolation of gibberellin responsive genes is difficult when whole plants or their slices are used.

### Disclosure of the Invention

The present invention has been made under the above circumstances. An obj ective of this invention is to provide methods for comprehensive and convenient identification and isolation of gibberellin responsive genes.

To date, those skilled in the art had never considered that cultured plant cells can respond to gibberellins. However, cultured cells, which are uniform and easy to handle, are suited for the purpose of comprehensive and convenient identification and isolation of gibberellin responsive genes. Considering the advantages of cultured cells, the present inventors developed methods for identifying and isolating gibberellin responsive genes using cultured cells. Specifically, the inventors first tested whether any change in gene expression was detected on treating suspension-cultured cells of rice with a gibberellin, GA3. No change in gene expression was detected. To maintain cell division activity, media for plant cell culture contains auxins that are not easily metabolized in plants. Therefore, considering the possibility that auxins might suppress gibberellin's action, the present inventors examined whether any change in gene expression was detected after cells were pre-cultured in a medium lacking auxins. In a surprising result, changes in the gene expression of several genes were detected shortly after gibberellin treatment.

"Gibberellin" is a general term for compounds that comprise an *ent*-gibberellane backbone. Only biologically active gibberellins were effective in the induction of expression of these genes. This strongly suggested that the induction of expression of these genes was mediated by signal transduction through a gibberellin receptor. Results of analysis using inhibitors of protein kinase and protein phosphatase suggested the involvement of protein kinase and protein phosphatase in the process of signal transduction leading to gene expression. In addition, a correlation was recognized between gibberellin-dependent gene expression in cultured cells and in the green leaves of whole plants. These results show that gibberellin responsive genes in whole plants can be identified and isolated using cultured cells.

In other words, for the first time in the world, the present inventors have succeeded in developing methods for identifying and isolating gibberellin responsive genes using cultured plant cells, thereby accomplishing the present invention. More specifically, the present invention provides:
[1] a method for identifying gibberellin responsive genes, wherein the method comprises the steps of:
   (a) contacting cultured plant cells with gibberellin,
   (b) detecting gene expression in the cultured plant cells contacted with gibberellin, and
   (c) identifying genes that have changes in expression when compared with cells that were not contacted with gibberellin;
[2] a method of screening for gibberellin responsive genes, wherein the method comprises the steps of:
   (a) contacting cultured plant cells with gibberellin,
   (b) detecting gene expression in the cultured plant cells contacted with gibberellin,
   (c) identifying genes that have changes in expression when compared with cells that were not contacted with gibberellin, and
   (d) isolating the genes identified in step (c);
[3] a method for determining the presence or absence of gibberellin responsiveness in a test gene, wherein the method comprises the steps of:
   (a) contacting cultured plant cells with gibberellin, and
   (b) measuring the expression of a test gene in the cultured plant cells contacted with gibberellin,
   wherein the test gene is judged to be gibberellin responsive when its expression is different from that in cells not contacted with gibberellin; and
[4] the method according to any one of [1] to [3], wherein plant cells cultured in a medium without auxins are used.

The present invention provides methods for identifying gibberellin responsive genes using cultured plant cells. In the methods for identification of this invention, the cultured plant cells are first contacted with gibberellin.

In this invention, "gibberellin responsive genes" means genes that show gibberellin-dependent changes in expression. Herein, "changes in expression" means an increase or decrease in expression. An increase or decrease in expression includes a transient increase or decrease. Decrease of expression includes complete blocking of expression.

In this invention, the term "gibberellins" refers to four-membered ring diterpenoid carboxylic acids comprising a basic *ent*-gibberellane structure. To date, about 120 different chemical species of gibberellin are known, including GA1, GA3, GA4, GA13, GA17, GA18, GA19, GA20, GA21, and GA22. In this invention, gibberellins include those described above; however, they are not limited thereto.

When the gibberellins of this invention are natural type gibberellins, they can be obtained by purification from desired plants, according to known methods in the art. In the cases of GA1, GA3, and GA4, products commercially available from Wako Pure Chemicals can be used (catalogue Nos. 077-04191, 075-02811, and 072-02821). In addition, methods for preparing synthetic gibberellins are well known to those skilled in the art.

The plant species from which the cultured cells used in this invention are derived are not particularly limited. Examples of plants include useful agricultural crop plants such as cereals, vegetables and fruit trees, and horticultural plants such as ornamental foliage plants. More specifically, rice, corn, wheat, barley, rapeseed, soybean, cotton, tomato, potato, chrysanthemum, rose, carnation, and cyclamen can be employed as examples. Preferably, the plant is a gramineous plant, and most preferably one being rice.

The methods for preparing the cultured plant cells of this invention are not particularly limited. For example, cultured cells of rice (mostly cv. Nipponbare) can be obtained by incubating dehulled seeds (husked rice) on agar medium (1% agar) containing 2 mg/L of 2,4-D (2,4-dichlorophenoxyacetic acid) in commercially available MS medium, kept in the dark at 25°C for about two weeks to form calluses, followed by successively culturing these calluses in liquid L medium (similar to N6 medium) containing 2,4-D (1mg/L). Cells are transferred to new medium once a week and containers are rotated at 120 rpm at 25°C. The above-mentioned L medium contains 2830 mg/L of KNO₃, 463 mg/L of (NH₄)₂SO₄, 166 mg/L of CaCl₂•2H₂O, 185 mg/L of MgSO₄•7H₂O, 400 mg/L of KH₂PO₄, 5.6 mg/L of FeSO₄•7H₂O, 7.5 mg/L of Na₂EDTA, 4.4 mg/L of MnSO₄•4H₂O, 1.5 mg/L of ZnSO₄•7H₂O, 0.8 mg/L of KI, 1.6 mg/L of H₃BO₃, 0.025 mg/L of CuSO₄•5H₂O, 0.25 mg/L of NaMoO₄•2H₂O, 0.025 mg/L of CoCl₂•6H₂O, 0.5 mg/L of nicotinic acid, 0.1 mg/L of thiamine-HCl, 0.5 mg/L of pyridoxine-HCl, 100 mg/L of myo-inositol, 2 mg/L of glycine, 1 mg/L of 2,4-D, and 30 g/L of sucrose. L medium can be prepared by using KOH to adjust the pH of this mixture to 5.8, and then autoclaving.

Furthermore, the culture conditions of the cultured plant cells are not particularly limited. Culture conditions suited to the desired plant cells can be selected as appropriate.

Prior to being used to detect gene expression, plant cells are pre-cultured in a medium that does not contain substances that inhibit response to gibberellin. Preferably, the medium does not contain auxins. In this invention, the term "auxin" refers to any one of the plant hormones that are synthesized in the tips of plant stems and that stimulate/regulate plant growth with their polar movement, including synthetic substances that show equivalent activities. In this invention, auxins are not particularly limited, and natural auxins such as indoleacetic acid, as well as synthetic auxins such as 2,4-dichlorophenoxyacetic acid and naphthaleneacetic acid can be employed as examples.

Contacting gibberellin with cultured plant cells is usually carried out by adding gibberellin to a medium for cultured plant cells, but the method of contact is not particularly limited as long as it can induce response to gibberellin in cultured plant cells.

In the method for identification of this invention, gene expression is detected in cultured plant cells that have been contacted with gibberellin.

"Gene expression" as used in this invention includes both transcription and translation. Methods for detection that use the amount of transcription product (mRNA amount) as an indicator include the DNA microarray method and the RNA display method; however, the methods are not limited thereto. In the DNA microarray method, gene expression can be detected by first preparing a single-stranded cRNA probe by reverse transcribing poly(A)-RNA extracted from cells, in the presence of cy5-dCTP, and then analyzing the results of hybridization using an array scanner (Microarray Scanner FLA8000 (Fujifilm)). IntheRNAdisplaymethod, geneexpressioncanbedetected by using PCR with an appropriate primer to amplify reverse-transcripts of the extracted RNA, and then comparing the products.

Methods for detection using the amount of translation product as an indicator are not particularly limited. For example, protein-containing fractions can be collected according to standard methods well known in the art and the expression of these proteins can be detected using electrophoretic methods such as SDS-PAGE and two-dimensional electrophoresis.

In the methods for identification of this invention, genes that show a change in expression compared to when there is no contact with gibberellin (a control) are identified. When the expression of a certain gene is increased compared to that of a control, the gene is identified as a gene whose expression is induced in response to gibberellin. On the other hand, when the expression of a certain gene is decreased compared to that of a control, the gene is identified as a gene whose expression is suppressed in response to gibberellin.

Furthermore, this invention provides methods of screening for gibberellin responsive genes. These screening methods isolate the genes identified by the above-described methods. Methods for isolating genes are not particularly limited. For example, when the DNA microarray method is used to detect gene expression, the desired gene can be isolated by obtaining a clone (*e*.*g*., from a gene bank) that corresponds to spots judged to be induced. When the RNA display method is used, bands judged to be amplified are cut from the gel, amplified by PCR, and then cloned. Full-length cDNA chains can then be obtained by screening a library. Induction of gibberellin-dependent expression of an isolated gene is preferably confirmed using methods such as Northern hybridization.

The present invention also provides methods for determining the presence or absence of gibberellin responsiveness in test genes.

In the methods for determination of this invention, cultured plant cells are first contacted with gibberellin. The test genes are notparticularlylimited. Anygeneforwhichevaluationofthepresence or absence of gibberellin responsiveness is desired can be used as a test gene. The source and preparation of cultured plant cells, chemical species of gibberellin, and the contact of gibberellin with cultured plant cells are similar to those described above in the identification methods of this invention.

In the methods for determination of this invention, expression of test genes is then measured in the cultured plant cells contacted with gibberellin.

Expression of a test gene can be detected at either the transcriptional or translational level, as in the identification methods of the present invention. Detection at the transcriptional level can be carried out as follows: Standard methods can be used to extract mRNA from cultured cells contacted with gibberellin, Northern hybridization can then be carried out on this mRNA, using as the probe a polynucleotide that hybridizes to the mRNA corresponding to the test gene. RT-PCR method can also be applied, wherein the mRNA extracted as described above is used as a template, and a pair of oligonucleotides that hybridize to the mRNA corresponding to the test gene is used as the primers to carry out the polymerase chain reaction.

Detection at the translational level can be carried out by Western blotting using an antibody against a protein encoded by the test gene, and by analyzing the positions of spots in two-dimensional electrophoresis, etc. Antibodies for detection can be prepared by a method known to those skilled in the art.

In the methods for determination of this invention, when the expression of a test gene is changed compared to when there is no gibberellin contact (a control), the gene is judged to possess gibberellin responsiveness. In other words, when the expression of a test gene is increased compared to that of a control, the gene is identified as a gene whose expression is induced in response to gibberellin. On the other hand, when expression of a test gene is decreased compared to that of a control, the gene is identified as a gene whose expression is suppressed in response to gibberellin.

### Brief Description of the Drawings

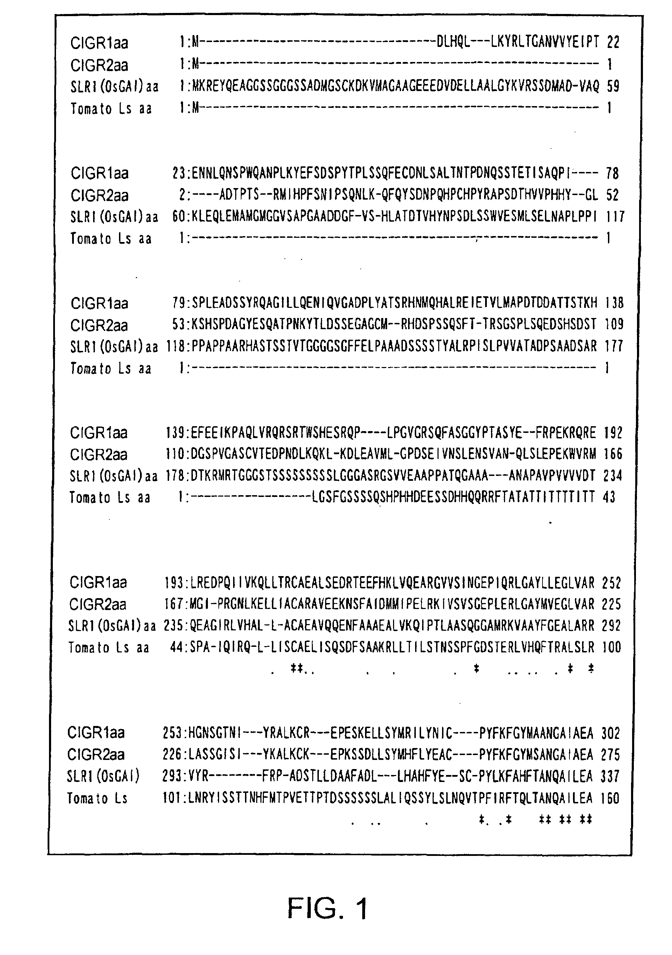

Fig. 1 depicts a comparison of the amino acid sequences encoded by the CIGR1 gene and CIGR2 gene with those of the GRAS family. SLR1 (OsGAI) indicates the rice gibberellin signal repressor (SEQ ID NO: 5) and Tomato Ls indicates the tomato lateral bud suppressor (SEQ ID NO: 6). Amino acid residues conserved in all four sequences are indicated by *, and amino acid residues conserved in three sequences are indicated by •.

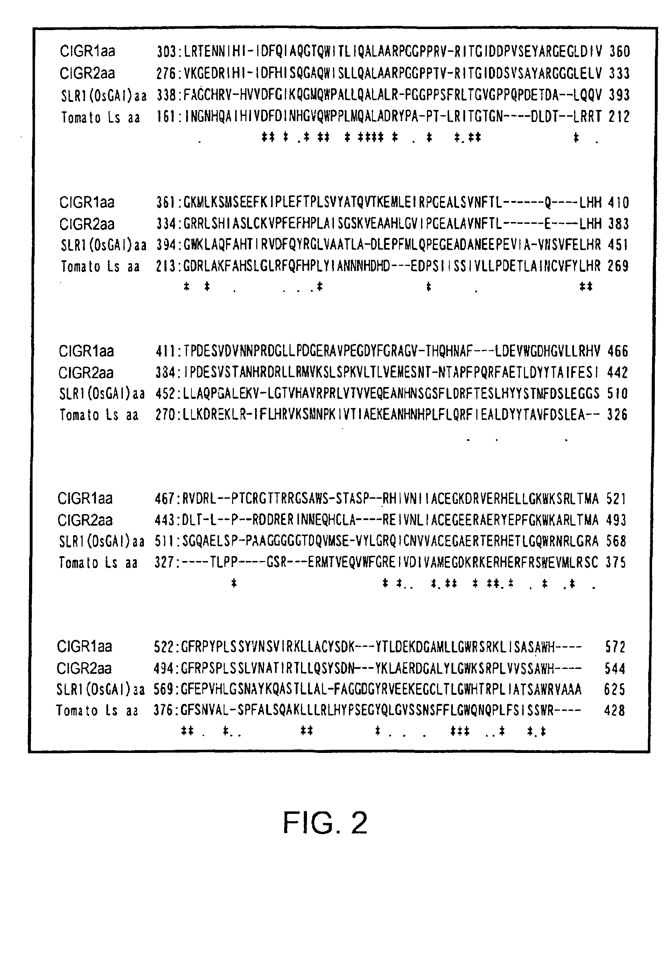

Fig. 2 is the continuation of Fig. 1.

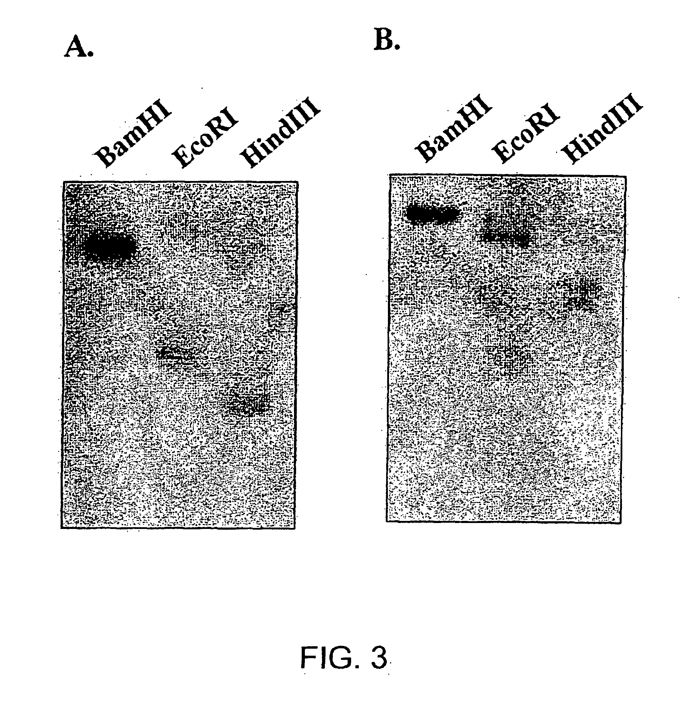

Fig. 3 depicts photographs showing the results of genomic Southern hybridization of the CIGR1 gene and CIGR2 gene. A: CIGR1 gene; B: CIGR2 gene.

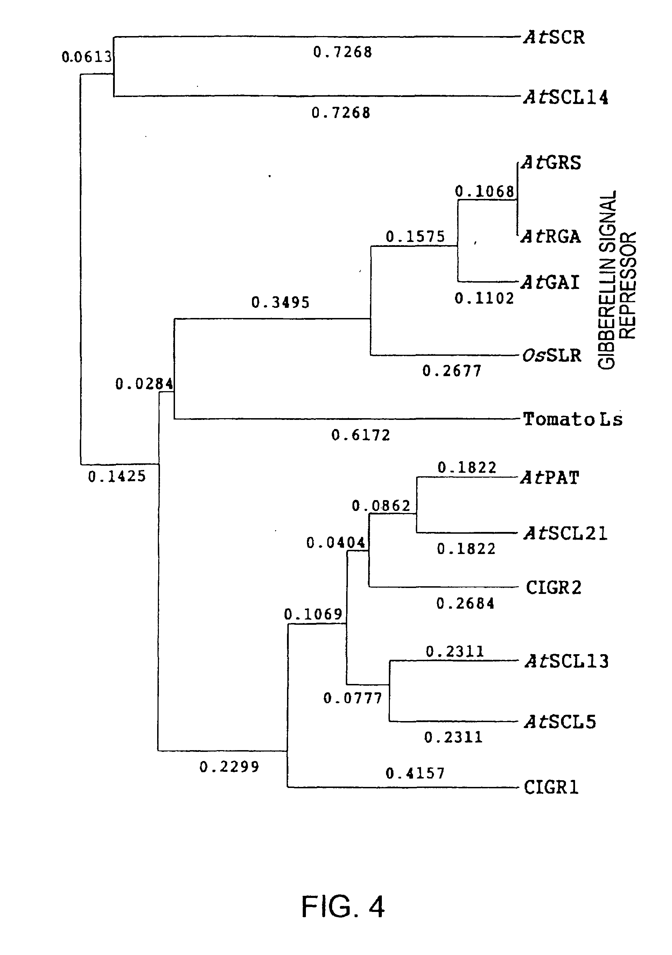

Fig. 4 shows the phylogenetic relationship of the CIGR1 gene and CIGR2 gene at the amino acid level. AtSCR: Arabidopsis SCARECROW; AtSCLn: SCARECROW-like genes; AtGRS: an AtGAI-like gene of unknown function; AtGRA: an Arabidopsis gibberellin signal repressor; AtGAI: an Arabidopsis gibberellin signal repressor (functional share with GRA is unknown); OsSLR: rice gibberellin signal repressor; Tomato Ls: tomato lateral bud suppressor; AtPAT: Arabidopsis light signal transduction factor; AtSCL21: an Arabidopsis SCARECROW-like gene (function unknown) ; CIGR2 : a rice gene reported in this study; AtSCL13: an Arabidopsis SCARECROW-like gene (function unknown); AtSCL5: an Arabidopsis SCARECROW-like gene (function unknown); CIGR1: a rice gene reported in this study.

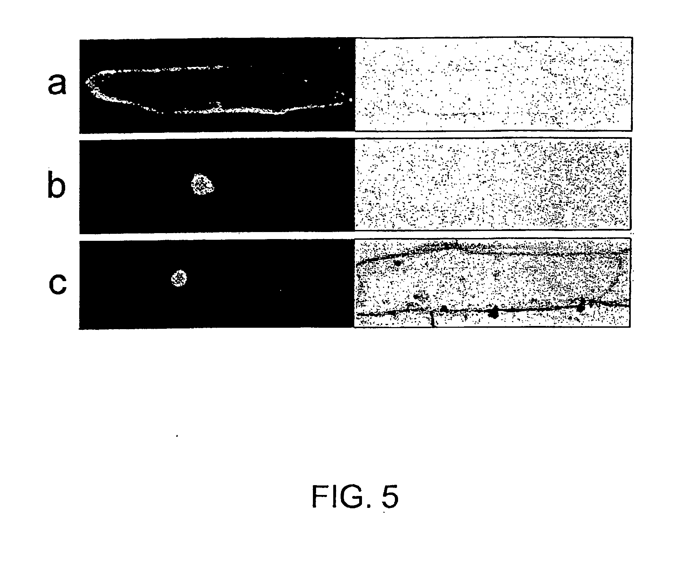

Fig. 5 depicts photographs representing the nuclear localization of the CIGR1 gene and CIGR2 gene. A fused gene 35S/CIGR1/GFP or 35S/CIGR2/GFP was introduced into onion epidermal cells using particle gun method. A laser confocal microscope was used for observations. The fused gene 35S/GFP was used as a control. a: 35S/GFP; b: 35S/CIGR1/GFP; c: 35S/CIGR2/GFP.

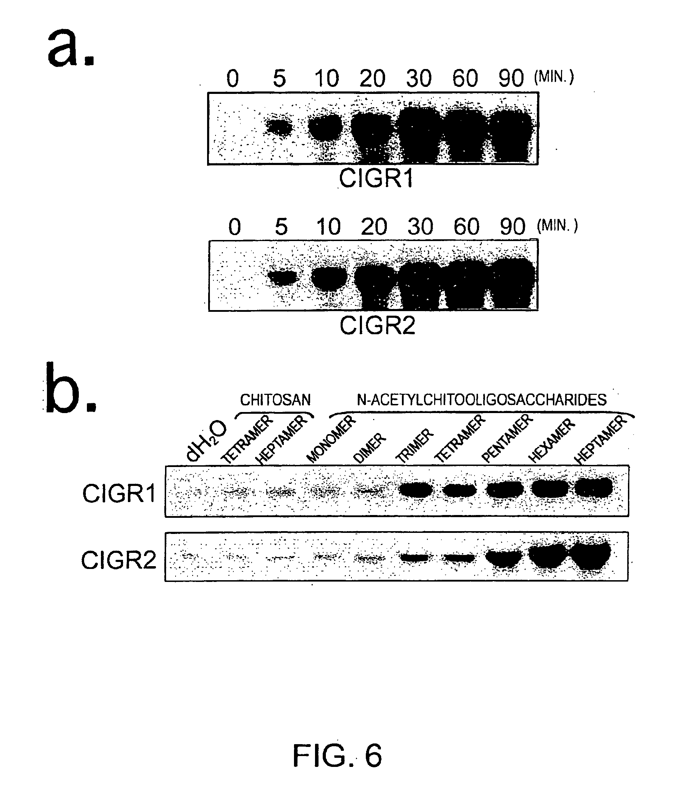

Fig. 6 depicts photographs representing CIGR1 gene and CIGR2 gene responsiveness to chitin oligomers. a: time-courses (in minutes) of expression induced by chitin heptamer treatment. b: induction activities of chitin oligomers and chitosan oligomers.

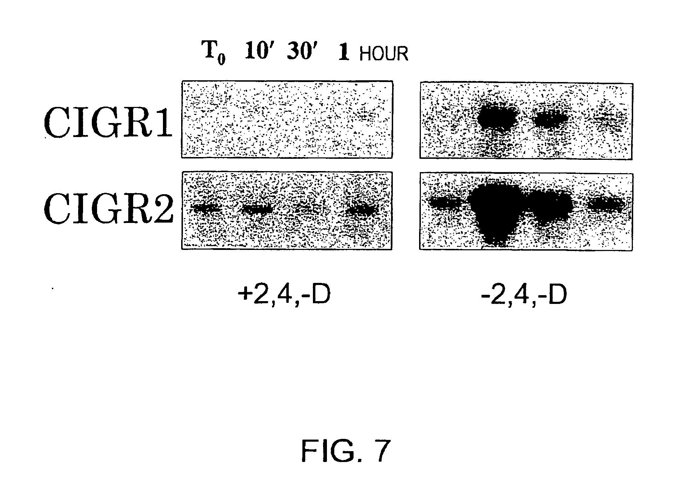

Fig. 7 depicts photographs representing the effect of 2,4-D on the responses of the CIGR1 gene and CIGR2 gene to GA3. The times indicated are times after GA3 treatment.

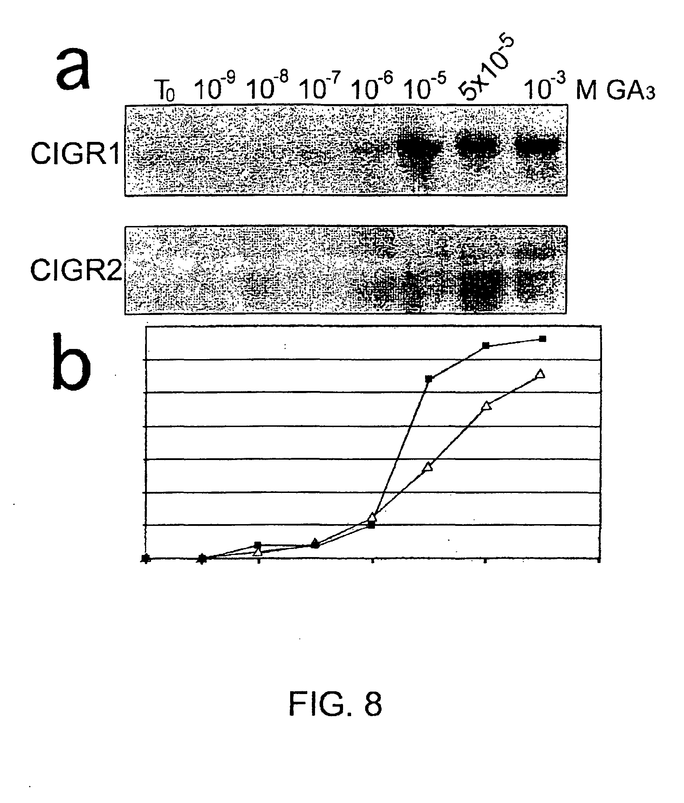

Fig. 8 depicts photographs and a diagram representing the effect of GA3 concentration on the induction of expression of the CIGR1 gene and CIGR2 gene. a: photographs representing the results of Northern blot hybridization analysis of total RNA extracted after ten minutes of GA3 treatment at various concentrations (expressed in M). b: diagram representing the results of quantitative determination of signals in 'a' using an image analyzer. Squares indicate the CIGR1 gene. Open triangles indicate the CIGR2 gene.

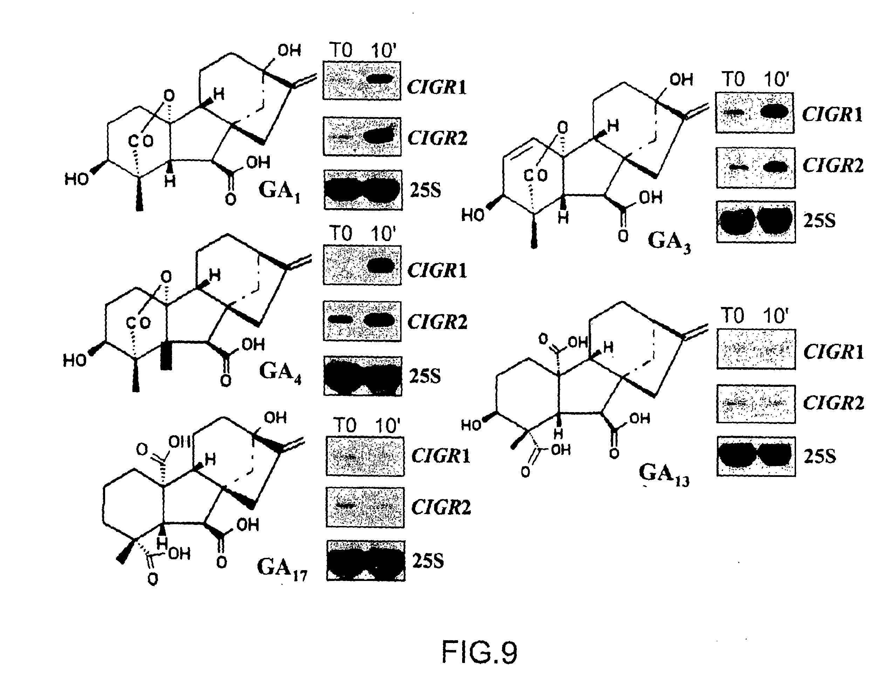

Fig. 9 depicts diagrams and photographs representing the physiological activities of gibberellins and expression of genes. Total RNA was extracted after treating cultured rice cells with active gibberellin species (GA1, GA3, or GA4) or inactive gibberellin species (GA13 or GA17) for ten minutes. The photographs show the results of analysis by Northern blot hybridization.

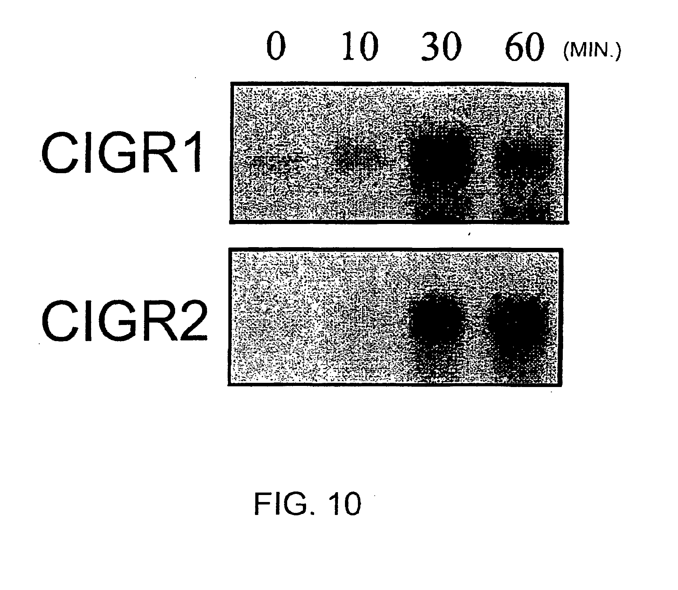

Fig. 10 depicts photographs representing the expression of the CIGR1 gene and CIGR2 gene in green leaves of rice after GA3 treatment. After spraying rice plants with GA3, the third and fourth leaves were sampled at certain intervals. Total RNA extracted from the third and fourth leaves was analyzed using Northern blot hybridization.

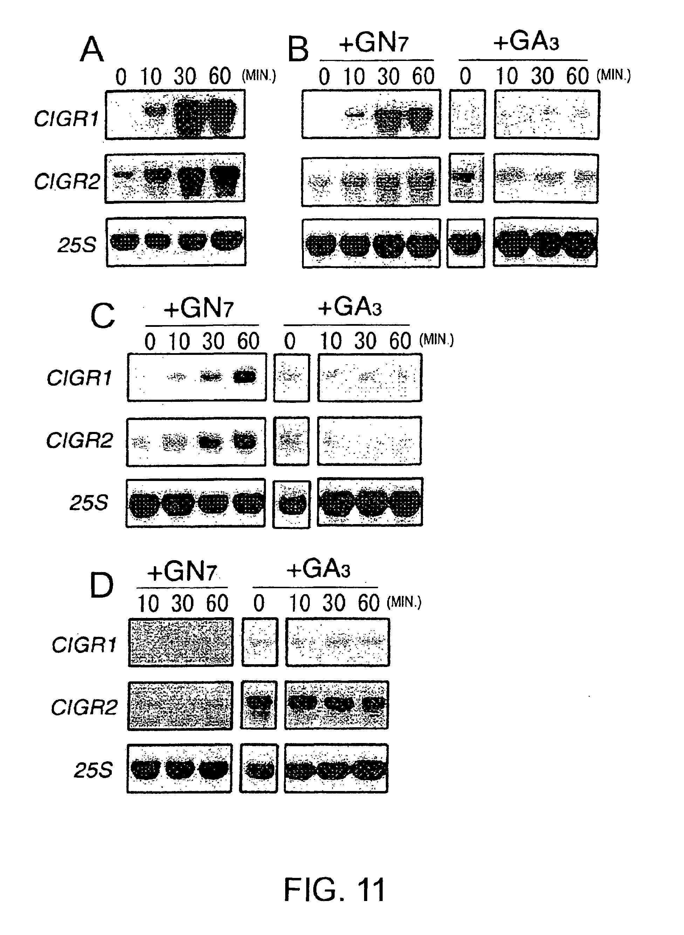

Fig. 11 depicts photographs representing the effects of protein kinase inhibitors on elicitor response and gibberellin response. The following compounds were added ten minutes before treatment with chitin heptamer (GN7) or gibberellin (GA3). (A): the positive elicitor response control; (B): okadaic acid (1 µM); (C): lavendustin A (30 µM); (D): K-252a (20 µM).

### Best Mode for Carrying out the Invention

Herein below, the present invention will be specifically described with reference to Examples; however, it is not to be construed as being limited thereto. The Examples were carried out using the materials and methods described below.
(1) Rice cultured cells: The cultured cells of rice were induced from germinating rice seeds *(Oryza sativa* cv. Nipponbare) on an N6 agar plate containing 1 ppm 2,4-D (2, 4-dichlorophenoxyacetic acid) . The cells were successively cultured in liquid N6 medium as follows: Once a week, cells of about 1 ml in volume were transferred to 150 ml of N6 medium. Every two weeks, cell clusters were passed through a 20-mesh metal screen to reduce their size. Cells which had been transferred to 30 ml of medium with no screen treatment, and then shake-cultured for four to six days, were used for extraction of RNA for Northern blotting.
(2) RNA extraction: The extraction of total RNA from cultured cells and green leaves of rice was carried out according to the phenol-SDS method. A Polytron homogenizer was used to homogenize living tissue in the presence of about tenfold or more volume to tissue-volume of phenol (about 90% saturated with water) , and a volume of buffer solution (50 mM Tris-HCl pH 9.0, 1% SDS, 50 mM NaCl) equal to this phenol volume. The aqueous phase obtained by centrifugation was then repeatedly extracted with phenol. Total nucleic acid was precipitated by adding 0.6 volume of isopropanol to the extract and stirring the mixture. After dissolving the precipitate in water, a 0.25 volume of 10M LiCl was added, and the mixture was cooled on ice. High molecular weight RNA was collected by centrifugation, washed with 70% ethanol, and then dissolved in a small volume of water to be used as an RNA sample.
(3) A method for gibberellin treatment of cultured cells and green leaves: The cultured cells were pre-washed several times with 2, 4-D-free N6 medium, and then suspended in the same medium and shaken for two hours at 25°C. Gibberellin (GA3 dissolved in ethanol) was added to make up each final concentration, cells were cultured with shaking for predetermined periods, and then used for RNA extraction. Nipponbare plants grown for three weeks after seeding were sprayed withgibberellin. The thirdand fourth leaves were collected at certain intervals,and totalRNA wasextracted. Elicitor treatment was carried out by directly adding aqueous elicitor solution to cultured cells of rice.
(4) Hybridization: RNA for Northern hybridization was denatured using the glyoxal method. 2.7 µl of glyoxal (final concentration, 1M) , 1.6 µl of sodium phosphate (pH 7.0, final concentration, 10 mM), and 8 µl of dimethylsulfoxide (final concentration, 50%) were added to 10 µg of total RNA (3.7 µl). The mixture was kept at 50°C for one hour and then subjected to electrophoresis in 1.4% agarose (10 mM sodium phosphate, pH 7.0). After electrophoresis, RNA was blotted onto a nylon membrane (Biodyne A). The RNA was fixed onto the membrane by treatment for two hours at 80°C. Hybridization was carried out in a mixture of 50% formamide, 0.1% SDS, 0.1 mg/ml salmon sperm DNA, 5x SSPE (0.9 M NaCl, 50 mM sodium phosphate, 5 mM EDTA, pH 7.4) , and 5x Denhardt's solution (0.1% bovine serum albumin, 0.1% Ficoll, 0.1% polyvinylpyrrolidone) at 42°C for one day. The membrane was then washed with 0.1 x SSC (15 mM NaCl, 1.5 mM sodium citrate) three times for five minutes each time at room temperature, and twice for 30 minutes each time at 65°C, and then exposed on x-ray film.
(5) DNA microarray analysis: Microchips (http://cdna01.dna.affrc.go.jp/RMOS/index.html) were prepared using rice ESTs (1265 clones) obtained from the Rice Genome Project. Single-stranded cDNA probes were prepared by reverse transcribing poly(A)-RNA extracted from cells that did not undergo elicitor treatment, and from those that underwent a 15-minute elicitor treatment in the presence of cy5-dCTP. The results were analyzed using Microarray Scanner FLA8000 (Fujifilm).

### [Example 1]

Chitin oligomers (N-acetylchitooligosaccharides) are a major component of the cell wall of rice blast disease fungus, and at low concentrations induce various defense responses in cultured cells of rice. In other words, these substances are known to act as powerful elicitors (substances that induce biological defense responses) (Yamada, A., Shibuya, N., Kodama, O., and Akatsuka, T., "Induction of phytoalexin formation in suspension-cultured rice cells by *N*-acetylchitooligosaccharides", Biosci. Biotech. Biochem. (1993) 57, 405-409). In the process of chitin oligomer action, known defense-related enzyme genes, PAL, chitinase, and glucanase, have been shown to be expressed (He, D.-Y., Yazaki, Y., Nishizawa, Y., Takai, R., Yamada, K., Sakano, K., Shibuya, N., and Minami, E., "Gene activation by cytoplasmic acidification in suspension-cultured rice cells in response to the potent elicitor, *N*-acetylchitoheptaose", Mol. Plant-Microbe Int. (1998) 12, 1167-1174; Nishizawa, Y., Kawakami, A., Hibi, T., He, D.-Y., Shibuya, N., and Minami, E., "Regulation of the chitinase gene expression in suspension-cultured rice cells by *N*-acetylchitooligosaccharides: differences in the signal transduction pathways leading to the activation of elicitor-responsive genes", Plant Mol. Biol. (1999) 39, 907-914) . In addition, using more rapid time-courses, three novel early genes, EL2, EL3, and EL5, were found to be expressed (Minami, E., Kuchitsu, K., He, D.-Y., Kouchi, H. , Midoh, N., Ohtsuki, Y., and Shibuya, N., "Two novel genes rapidly and transiently activated in suspension-cultured rice cells by treatment with *N*-acetylchitoheptaose, a biotic elicitor for phytoalexinproduction", Plant Cell Physiol. (1996) 37, 563-567; Takai, R., Hasegawa, K., Kaku, K., Shibuya, N., and Minami, E., "Isolation and analysis of expression mechanisms of a rice gene, EL5, which shows structural similarity to ATL family from Arabidopsis, in response to *N*-acetylchitooligosaccharide elicitor", Plant Sci. (2001) 160, 577-583).

From the MAFF Genebank, the present inventors obtained two ESTs (c72495 and AU094860) whose DNA microarray signals were significantly increased after 15-minutes of elicitor treatment, and determined their entire nucleotide sequences. These deduced amino acid sequences were searched for homology with known sequences. The search results indicated that the two sequences comprised homology with known GRAS family genes (Fig. 1 and Fig. 2).

The GRAS family takes its name from the capital letters of its members; a gene which regulates the formation of radial structures of *Arabidopsis* roots and stems and is assumed to be a transcription factor (SCARECROW), and two genes that down regulate gibberellin signals in Arabidopsis (GAI and RGA) (Di Laurenzio, L. , Wysocka-Diller, J., Malamy, J. E., Pysh, L., Helariutta, Y., Freshour, G., Hahn, M. G., Feldmann, K. A. and Benfey, P. N. , "The SCARECROW gene regulated an asymmetric cell division that is essential for generating the radial organization of the Arabidopsis root", Cell (1996) 86, 423-433; Peng, J., Carol, P., Richards, D. E., King, K. E., Cowling, R. J., Murphy, G. P., and Harberd, N. P., Genes and Development, (1997) 11, 3194-3205; Silverstone, A. L. , Ciampaglio, C. N., and Sun, T.-P. , "TheArabidopsis RGA gene encodes a transcriptional regulator repressing the gibberellin signal transduction pathway", The Plant Cell (1998) 10, 155-169; Pysh, L. D., Wysocka-Diller, J. W. , Camilleri, C., Bouchez, D., and Benfey, P. N., "The GRAS gene family in Arabidopsis: sequence characterization and basic expression analysis of the SCARECROW-LIKE genes", The Plant J. (1999) 18, 111-119). The amino acid sequences of the GRAS family are characterized by N-terminal regions with low homology, a leucine heptad structure, VHIID domain, etc. These structural characteristics are not known in animals and microorganisms. They are unique to plants.

The two genes induced by elicitor treatment comprised VHIID domains and C-terminal amino acid sequences that are highly conserved among SCARECROW and its family. However, they did not comprise the typical leucine heptad structure. Also absent was the DELLA sequence, which is characteristic of gibberellin signal repressors and plays important roles in catching the gibberellin signal (Harberd, N. P., King, K. E., Carol, P., Cowling, R. J., Peng, J., and Richards, D. E., BioEssays (1998) 20, 1001-1008). Hereafter, C72495 is referred to as the CIGR1 gene (its nucleotide sequence and amino acid sequence are shown in SEQ ID NOs : 1 and 2, respectively) , and AU94860 is referred to as the CIGR2 gene (its nucleotide sequence and amino acid sequence are shown in SEQ ID NOs: 3 and 4, respectively). These two genes are 57% homologous at the nucleotide level and 40% homologous at the amino acid level. The results of genomic Southern hybridization suggest that a single copy of each of these genes is present (Fig. 3).

Fig. 4 depicts the estimated molecular phylogenetic tree of the CIGR1 and CIGR2 gene products and those of the GRAS family gene products with known structures. CIGR2 is thought to be somewhat closer to Arabidopsis AtSCL5 than to CIGR1. It is also suggested that gibberellin signal repressors form a single subfamily, and that rice OsGAI (SLR) is closer to the corn repressor and Arabidopsis repressor than to the CIGR1 gene and CIGR2 gene.

### [Example 2]

The gene products of the GRAS family are regarded as transcriptional regulators, but their involvement in the regulation of gene expression is unknown. Arabidopsis SCARECROW does not comprise the typical nuclear localization signal. However, it is estimated to be a transcription factor because its N-terminal region is rich in serine, threonine, proline, and glutamine. The GAI gene, which is an Arabidopsis gibberellin signal repressor, and the GRS gene, which is very similar to that, comprise sequences like nuclear localization signals (Peng, J., Carol, P., Richards, D. E., King, K. E., Cowling, R. J., Murphy, G. P., and Harberd, N. P., Genes and Development (1997) 11, 3194-3205). Furthermore, experiments using a GFP-fusion protein showed that RGA, another repressor, was localized in the nucleus (Silverstone, A. L. , Ciampaglio, C. N. , and Sun, T-P., "The Arabidopsis RGA gene encodes a transcriptional regulator repressing the gibberellin signal transduction pathway", The Plant Cell (1998) 10, 155-169). To analyze the intracellular localization of the CIGR1 and CIGR2 genes, the particle gun method was used to introduce chimeric plasmids whose translation regions were connected in-frame to GFP. The behavior of the fusion proteins was monitored using GFP fluorescence, and an image of nuclear localization of a CIGR2-GFP fusion protein was observed (Fig. 5). Since this kind of image was not observed when only GFP was introduced, it was concluded that the product of CIGR2 gene was localized in the nucleus.

### [Example 3]

Both the CIGR1 gene and CIGR2 gene were identified as elicitor responsive genes using DNA microarray analysis. The elicitor responsiveness of these two genes was analyzed using Northern blot hybridization. Significant increases in mRNA amount were observed in both genes five minutes after treatment with chitin heptamers. Expression continued to increase for up to 90 minutes (Fig. 6a). It has been reported that the elicitor activity of chitin oligomers in rice depends on their size; that is, chitin heptamers and octamers comprise the strongest effects, and chitosan oligomers that are deacetylated derivatives comprise very low activities. Therefore, the present inventors studied the induction of expression of these two genes when cells were treated with chitin oligomers from monomers to heptamers, and tetramer and heptamer chitosan oligomers. Both genes showed the strongest responses to chitin heptamers. They showed no significant response to chitosan oligomers (Fig. 6b).

### [Example 4]

Ashikari *et al*. (Ashikari, M., Wu, J., Yano, M., Sasaki, T., and Yoshimura, A., "Rice gibberellin-insensitive dwarf mutant gene Dwarf 1 encodes the alpha-subunit of GTP-binding protein", Proc. Natl. Acad. Sci. U. S. A. (1999) 96, 10284-10289) and Fujisawa *et al*. (Fujisawa, Y., Kato, T., Ishikawa, A., Kitano, H., Sasaki, T., Asahi, T., and Iwasaki, Y., "Suppression of the heterodimeric G protein causes abnormal morphology, including dwarfism, in rice", Proc. Natl. Acad. Sci. U. S. A. (1999) 96, 7575-7580) both independently discovered that gene D1, which causes dwarf mutant d1 in rice, encodes the α-subunit of the trimeric G protein. In animal cells, trimeric G proteins are known to couple with seven-transmembrane receptors and to play important roles in the transduction of extracellular information (Neer, E. J., "Heterotrimeric G proteins: organizers of transmembrane signals", Cell (1995) 50, 1011-1019).

Analysis using inhibitors and activators suggested that the G proteins were involved in the transduction of elicitor signals (Legendre, L., Heinsyein, P. F., and Low, P. S., "Evidence for participation of GTP-binding proteins in elicitation of the rapid oxidative burst in cultured soybean cells", J. Biol. Chem. (1992) 267, 20140-20147). However, some problems were pointed out, such as the specificity of these inhibitors was not always precise (Ephritikhine, G., Pradier, J.-M., and Guern, J., "Complexity of GTP γS binding to tobacco plasma membranes", Plant Physiol. Biochem. (1993) 31, 573-584). Thus, no clear conclusion was obtained concerning the involvement of the D1 gene product.

Tsukada *et al*. compared in detail the various elicitor responses of calluses derived from d1 strain seeds with those of the wild type, demonstrating that there was no significant difference between them (Tsukada, K., Ishizaka, M., Fujisawa, Y., Iwasaki, Y., Yamaguchi, T., Minami, E., and Shibuya, N., "Rice receptor for chitin oligosaccharide elicitor does not couple to heterotrimeric G-protein: Elicitor responses of suspension cultured rice cells from Daikoku dwarf (d1) mutants lacking a functional G-protein α-subunit", Physiol. Plantrum (2002) 116, 373-382).

Preliminary experiments demonstrated that the time-courses of CIGR1 gene and CIGR2 gene elicitor induction in the d1 strain were similar to those in the wild type.

On the other hand, Ueguchi-Tanaka *et al*. showed that the D1 gene is involved in the transduction of gibberellin signals, using the induction of α-amylase in aleurone in germinating seeds as an indicator (Ueguchi-Tanaka, M., Fujisawa, Y., Kobayashi, M., Ashikari, M., Iwasaki, Y., Kitano, H., and Matsuoka, M., "Rice dwarf mutant d1, which is defective in the α subunit of the heterotrimeric G protein, affects gibberellin signal transduction", Proc. Natl. Acad. Sci. U. S. A. (2000) 97, 11638-11643). Prior to this study, Schumacher *et al*. showed that the tomato lateral bud suppressor gene product belonged to the same gene family as SCARECROW (Schumacher, K., Schmitt, T., Rossberg, M., Schmitz, G., and Theres, K., "The Lateral suppressor (Ls) gene of tomato encodes a new member of the VHIID protein family", Proc. Natl. Acad. Sci. U. S. A. (1999) 96, 290-295). They pointed out the possibility of an interaction between this gene product and gibberellin in the discussion on its role in morphogenesis.

Thus, the present inventors examined the gibberellin responsiveness of the CIGR1 and CIGR2 genes. Suspension-cultured cells were treated with GA3, an active-type gibberellin, and changes in the expression of these two genes were analyzed over time. Expression of both genes was observed for three hours after treatment, and no significant change in expression amount was found. To maintain the cell's ability to divide, the medium used to culture plant cells comprised 2,4-D, auxins that are not easily metabolized by plants. It was thought that 2,4-D might suppress the action of gibberellin. Therefore, cells were pre-washed with 2,4-D-free medium, and treated with GA3 after two hours of pre-culture in the same medium. Transient expression with a maximum at ten minutes after treatment was observed (Fig. 7).

In the prior art, studies on the transduction of gibberellin signals were carried out mostly by analyzing α-amylase induction in the aleurone tissues of cereal seeds. The above-mentioned result is the first example using cultured cells. Responsiveness to gibberellin was then analyzed in more detail by using the expressions of the CIGR1 gene and CIGR2 gene in cultured cells as indicators.

Fig. 8 shows the effect of GA3 concentration on the induction of expression of these two genes. The induction of expression of both genes began to appear with GA3 treatment at 10⁻⁶ M. Induction reached near saturation at 10⁻⁴ M. Vishnevetsky *et al*. reported that CHRC, a carotenoid-associated protein in the chromoplasts of cucumber petals, required at least 10⁻⁷ M GA3 for induction by gibberellin, and that its expression level increased approximately linearly up to 10⁻⁴ M GA3 (Vishnevetsky, M., Ovadis, M., Itzhaki, H., and Vainstein, A., "CHRC, encoding a chromoplast-specific carotenoid-associated protein, is an early gibberellic acid-responsive gene", J. Biol. Chem. (1997) 272, 24747-24750). Though there were differences in the materials and the genes, they obtained similar results for effective GA3 concentrations.

### [Example 5]

Unlike auxins and cytokinins, gibberellins are compounds defined not by their physiological activities, but by their *ent*-gibberellane backbone. Therefore, there are large variations in their activities. In order to judge whether induction of CIGR1 gene and CIGR2 gene expression is based on physiological activity or on the *ent*-gibberellane backbone itself, induction of expression of these genes by active type gibberellins (GA1, GA3, and GA4) and inactive type gibberellins was analyzed (GA13 and GA17) (Crozier, A., Kuo, C. C., Durley, R. C., and Pharis, R. P., "The biological activities of 26 gibberellins in nine plant bioassays", Canadian J. Botany (1970) 48, 867-877.). The results showed that only active type gibberellins induced the expression of both genes (Fig. 9). This result strongly suggests that the above-mentioned induction of CIGR1 and CIGR2 gene expression by gibberellin occurs via signal transduction mediated by a gibberellin receptor.

### [Example 6]

In the presence of auxins, cultured plant cells are thought to be in an undifferentiated state. They also grow under heterotrophic conditions as they are devoid of chloroplasts. Due these and other factors, cultured cells are quite different from whole plants in their histological and physiological characteristics. Therefore, the possibility cannot be denied that the induction of CIGR1 and CIGR2 gene expression by gibberellin is a special phenomenon in cultured cells. Thus, the responses of these two genes to gibberellin were analyzed in the green leaves of rice. The third and fourth leaves of rice, three weeks after seeding, were sprayed with GA3 (50 µM) and total RNA was extracted at certain intervals to analyze changes in the expression level of these genes. Both genes showed very rapid transient expression with a maximum 30 minutes after spraying (Fig. 10). This strongly suggested that the signaling via a gibberellin receptor, found in cultured cells, was not peculiar to cultured cells, and that it also functioned in whole rice plants.

### [Example 7]

Kuo *et al*. found that the gibberellin' s induction of α-amylase in the aleurone layer of wheat was specifically inhibited by okadaic acid, a protein phosphatase inhibitor (Kuo, A., Cappelluti, S., Cervantes-Cervantes, M., Rodriguez, M., and Bush, D. S., "Okadaic acid, a protein phosphatase inhibitor, blocks calcium changes, gene expression and cell death induced by gibberellin in wheat aleurone cells", The Plant Cell (1996) 8, 259-269.). Okadaic acid is known to inhibit the animal protein phosphatases PP1 and PP2B.

On the other hand, the protein kinase inhibitors staurosporine and K252a hardly inhibited induction. Thus it was presumed that protein phosphorylation, especially by protein phosphatases, was critically involved in signal transduction from gibberellin to the α-amylase gene.

In contrast, our results to date suggest that the induction of expression of elicitor-responsive genes is strongly inhibited by K-252a pretreatment (He, D.-Y., Yazaki, Y., Nishizawa, Y., Takai, R., Yamada, K. , Sakano, K. , Shibuya, N. , and Minami , E. , "Gene activation by cytoplasm acidification in suspension-cultured rice cells in response to the potent elicitor, N-acetylchitoheptaose", Mol. Plant-Microbe Int. (1998) 12, 1167-1174; Nishizawa, Y., Kawakami, A., Hibi, T., He, D.-Y., Shibuya, N., and Minami, E., "Regulation of the chitinase gene expression in suspension-cultured rice cells by *N*-acetylchitooligosaccharides: differences in the signal transduction pathways leading to the activation of elicitor-responsive genes", Plant Mol. Biol. (1999) 39, 907-914).

The effects of various inhibitors on gibberellin and elicitor induction of the CIGR1 gene and CIGR2 gene in cultured rice cells were examined. With reference to the results of Kuo *et al*., cells pretreated with okadaic acid were tested. In these cells, induction of both genes by chitin heptamer was hardly inhibited, however induction by GA3 was inhibited almost completely (Fig. 11B). From these observations, the involvement of protein phosphatase in signal transduction from gibberellin to the CIGR1 and CIGR2 genes in cultured rice cells was presumed. Following these experiments, the effects of protein kinase inhibitors were studied. Lavendustin A, which is known as an inhibitor of receptor-type protein tyrosine kinase, exhibited a pattern of inhibition similar to that of okadaic acid. Lavendustin A hardly inhibited induction by chitin heptamer, but inhibited induction by GA3 almost completely (Fig. 11C). K-252-A, which is thought to inhibit both protein serine/threonine kinase and protein tyrosine kinase, almost completely inhibited the induction of both genes by chitin heptamer and GA3 (Fig. 11D).

α-amylase induction by gibberellin in the aleurone layer of wheat is reported to be inhibited by okadaic acid, but not by K-252-A (Kuo, A., Cappelluti, S., Cervantes-Cervantes, M., Rodriguez, M., and Bush, D. S. , "Okadaic acid, a protein phosphatase inhibitor, blocks calcium change, gene expression and cell death induced by gibberellin in wheat aleurone cells", The Plant Cell (1996) 8, 259-269).

The results shown in Figure 11 suggest that the induction of the CIGR1 and CIGR2 genes by gibberellin in cultured cells involves not only protein phosphatase, which is inhibited by okadaic acid, but also protein kinase, which is inhibited by K-252a, and lavendustin A. Signal transduction in cultured cells and in the aleurone layer had some aspects in common, and some aspects that were different. Signal transduction from gibberellins is thought to be qualitatively different to that from chitin heptamers.

Richards *et al*. proposed a hypothesis that the GRAS family corresponds to STATs, which are transcriptional factors found widely in metazoan organisms (Richards, D. E., Peng, J., and Harberd, N. P., BioEssays (2000) 22, 573-577). In STAT C-terminal regions, structures similar to the SH2 region are found, where the SH2 region is a structure common to the STAT family, which are transcription factors in animals. Near the C-terminal of the SH2 region, a tyrosine residue that is highly conserved in animal STATs and susceptible to phosphorylation, and an arginine residue that is supposed to interact electrostatically with the phosphorylated tyrosine, is conserved. Homology in amino acid sequence N-terminal regions is lower than in C-terminal regions. This is generally found in the GRAS family. The homology of the CIGR1 gene and CIGR2 gene at the nucleotide level was 57%. From the result of genomic Southern hybridization, it was estimated that these two genes were present in one copy each (Fig. 3). Five µg of rice genomic DNA was completely digested by restriction enzymes BamHI (B), EcoRI (E), or HindIII (H). The products were separated on agarose gel by electrophoresis, and transferred onto nitrocellulose membranes. Hybridization with ³²P-labelled CIGR1 and CIGR2 resulted in patterns consistent with the cDNA maps.

From these results, each of the full-length cDNA strands was thought to hybridize specifically with the corresponding gene product. Neither of the genes comprises the DELLA sequence, a common structure in the GAI/RGA subfamily, which are negative regulators of gibberellin signals.

### Industrial Applicability

The present invention made it possible to comprehensively and conveniently identify and isolate gibberellin responsive genes. In addition, the present invention made it possible to conveniently determine the presence or absence of gibberellin responsiveness in a certain gene. The gibberellin responsive genes identified by the methods of this invention are expected to have various applications, including in the generation of transgenic crops whose germination, elongation, flower bud formation, and so on has been modified.

## Claims

1. A method for identifying gibberellin responsive genes, wherein the method comprises the steps of:
(a) contacting cultured plant cells with gibberellin,
(b) detecting gene expression in the cultured plant cells contacted with gibberellin, and
(c) identifying genes that have changes in expression when compared with cells that were not contacted with gibberellin.

2. A method of screening for gibberellin responsive genes, wherein the method comprises the steps of:
(a) contacting cultured plant cells with gibberellin,
(b) detecting gene expression in the cultured plant cells contacted with gibberellin,
(c) identifying genes that have changes in expression when compared with cells that were not contacted with gibberellin, and
(d) isolating the genes identified in step (c).

3. A method for determining the presence or absence of gibberellin responsiveness in a test gene, wherein the method comprises the steps of:
(a) contacting cultured plant cells with gibberellin, and
(b) measuring the expression of a test gene in the cultured plant cells contacted with gibberellin,
wherein the test gene is judged to be gibberellin responsive when its expression is different from that in cells not contacted with gibberellin.

4. The method according to any one of [1] to [3] , wherein plant cells cultured in a medium without auxins are used.


SEQUENCE LISTING

<110> National Institute of Agrobiological Sciences

<120> METHODS FOR IDENTIFYING GIBBERELLIN RESPONSIVE GENES
USING CULTURED PLANT CELLS

<130> MOA-A0108P

<140>
<141>

<150> JP 2001-388993
<151> 2001-12-21

<160> 6

<170> PatentIn Ver. 2.1

<210> 1
<211> 2135
<212> DNA
<213> Oryza sativa L cv Nipponbare

<220>

<221> CDS

<222> (137)..(1852)

<400> 1
   gcacgagaga gaaccagaga gagagagaga gagagagaga gagagagagg agtggaagtg 60
   gatcggggca tcgctgcggc gtcggggctg tgcagatcgg aggaggtcat gaacgactaa 120
   tctccgttca tttatt atg gac ttg cac cag tta tta aag tac aga ttg act 172
   Met Asp Leu His Gln Leu Leu Lys Tyr Arg Leu Thr
   1 5 10
   ggc gct aac gtt gtg tac gaa att cct aca gag aac aac ttg caa aac 220
   Gly Ala Asn Val Val Tyr Glu Ile Pro Thr Glu Asn Asn Leu Gln Asn
   15 20 25
   tct ccc tgg caa gct aac cca ctg aag tac gag ttc agc gat tcc cca 268
   Ser Pro Trp Gln Ala Asn Pro Leu Lys Tyr Glu Phe Ser Asp Ser Pro
   30 35 40
   tac acc cct ctc tct tcc caa ttt gag tgt gac aat ttg tct gct ctt 316
   Tyr Thr Pro Leu Ser Ser Gln Phe Glu Cys Asp Asn Leu Ser Ala Leu
   45 50 55 60
   acc aac act cca gat aac cag agc tct aca gaa acc att tca gcc caa 364
   Thr Asn Thr Pro Asp Asn Gln Ser Ser Thr Glu Thr Ile Ser Ala Gln
   65 70 75
   cca atc tcc cca ttg gaa gca gac agc tca tat aga cag gcg ggt att 412
   Pro Ile Ser Pro Leu Glu Ala Asp Ser Ser Tyr Arg Gln Ala Gly Ile
   80 85 90
   ctt ctc cag gag aac att caa gtg gga gct gat ccg ttg tat gct aca 460
   Leu Leu Gln Glu Asn Ile Gln Val Gly Ala Asp Pro Leu Tyr Ala Thr
   95 100 105
   tca aga cat aat atg caa cac gcc tta cgg gag att gag act gtt ctg 508
   Ser Arg His Asn Met Gln His Ala Leu Arg Glu Ile Glu Thr Val Leu
   110 115 120
   atg gca cct gat aca gat gat gca aca act agc acc aag cat gag ttt 556
   Met Ala Pro Asp Thr Asp Asp Ala Thr Thr Ser Thr Lys His Glu Phe
   125 130 135 140
   gag gaa atc aag cct gct cag ctt gtg agg cag cgg tcg aga aca tgg 604
   Glu Glu Ile Lys Pro Ala Gln Leu Val Arg Gln Arg Ser Arg Thr Trp
   145 150 155
   agt cat gaa tca cgg cag ccg tta cct gga gtt ggt cgg tca cag ttt 652
   Ser His Glu Ser Arg Gln Pro Leu Pro Gly Val Gly Arg Ser Gln Phe
   160 165 170
   gca tct ggt gga tac ccc aca gca agc tat gaa ttt cgt cca gag aaa 700
   Ala Ser Gly Gly Tyr Pro Thr Ala Ser Tyr Glu Phe Arg Pro Glu Lys
   175 180 185
   cgg caa agg gag tta agg gaa gac cct cag ata att gtg aag cag cta 748
   Arg Gln Arg Glu Leu Arg Glu Asp Pro Gln Ile Ile Val Lys Gln Leu
   190 195 200
   tta acc agg tgt gct gag gct ctg agt gag gac agg aca gag gag ttc 796
   Leu Thr Arg Cys Ala Glu Ala Leu Ser Glu Asp Arg Thr Glu Glu Phe
   205 210 215 220
   cat aag ctt gtt cag gag gct cgt gga gtg gtc tca atc aac ggg gaa 844
   His Lys Leu Val Gln Glu Ala Arg Gly Val Val Ser Ile Asn Gly Glu
   225 230 235
   cca atc caa cgt cta ggt gct tac cta ctg gag ggt ttg gtt gct aga 892
   Pro Ile Gln Arg Leu Gly Ala Tyr Leu Leu Glu Gly Leu Val Ala Arg
   240 245 250
   cat gga aac tct ggc aca aac atc tac cgt gct ctg aag tgc cgt gag 940
   His Gly Asn Ser Gly Thr Asn Ile Tyr Arg Ala Leu Lys Cys Arg Glu
   255 260 265
   cca gag agc aag gag ctc ctg tcc tac atg aga att cta tac aat atc 988
   Pro Glu Ser Lys Glu Leu Leu Ser Tyr Met Arg Ile Leu Tyr Asn Ile
   270 275 280
   tgc cct tac ttc aag ttt ggc tat atg gca gcc aat ggg gcg att gca 1036
   Cys Pro Tyr Phe Lys Phe Gly Tyr Met Ala Ala Asn Gly Ala Ile Ala
   285 290 295 300
   gaa gca ttg aga act gag aac aat atc cac ata att gat ttt cag att 1084
   Glu Ala Leu Arg Thr Glu Asn Asn Ile His Ile Ile Asp Phe Gln Ile
   305 310 315
   gct caa ggg act caa tgg atc aca ctg ata caa gca tta gct gca agg 1132
   Ala Gln Gly Thr Gln Trp Ile Thr Leu Ile Gln Ala Leu Ala Ala Arg
   320 325 330
   cct ggt ggt cct cct cgt gtg cgg atc acc ggg ata gat gac cca gtg 1180
   Pro Gly Gly Pro Pro Arg Val Arg Ile Thr Gly Ile Asp Asp Pro Val
   335 340 345
   tca gag tat gct cgt ggt gaa ggt ctt gac att gtg ggg aaa atg ttg 1228
   Ser Glu Tyr Ala Arg Gly Glu Gly Leu Asp Ile Val Gly Lys Met Leu
   350 355 360
   aaa agc atg tct gaa gaa ttc aaa ata cct ctg gag ttt acg cct ctg 1276
   Lys Ser Met Ser Glu Glu Phe Lys Ile Pro Leu Glu Phe Thr Pro Leu
   365 370 375 380
   tct gtc tat gcc aca caa gtc acg aaa gag atg ctt gag atc agg cca 1324
   Ser Val Tyr Ala Thr Gln Val Thr Lys Glu Met Leu Glu Ile Arg Pro
   385 390 395
   ggt gaa gca ctg tct gta aac ttc aca ctc cag cta cac cac acc ccg 1372
   Gly Glu Ala Leu Ser Val Asn Phe Thr Leu Gln Leu His His Thr Pro
   400 405 410
   gac gag agc gtg gat gtc aac aac cca cgc gat ggt ctg cta ccg gat 1420
   Asp Glu Ser Val Asp Val Asn Asn Pro Arg Asp Gly Leu Leu Pro Asp
   415 420 425
   ggt gaa agg gct gtc ccc gaa ggt gac tac ttt ggt aga gca gga gtc 1468
   Gly Glu Arg Ala Val Pro Glu Gly Asp Tyr Phe Gly Arg Ala Gly Val
   430 435 440
   aca cac caa cac aac gcc ttt ctt gat gag gtt tgg gga gac cat gga 1516
   Thr His Gln His Asn Ala Phe Leu Asp Glu Val Trp Gly Asp His Gly
   445 450 455 460
   gta cta ctc cgc cat gtt cga gtc gat cga ctg cca acc tgc cgc ggg 1564
   Val Leu Leu Arg His Val Arg Val Asp Arg Leu Pro Thr Cys Arg Gly
   465 470 475
   aca aca agg aga gga tca gcg tgg agc agc act gcc tcg cca agg cac 1612
   Thr Thr Arg Arg Gly Ser Ala Trp Ser Ser Thr Ala Ser Pro Arg His
   480                 485                 490
   atc gtc aac atc atc gcc tgc gag ggg aag gac agg gtg gag agg cat    1660
   Ile Val Asn Ile Ile Ala Cys Glu Gly Lys Asp Arg Val Glu Arg His
           495                 500                 505
   gag ctg ctt ggc aag tgg aag tcg agg ctg acc atg gcc ggc ttc agg    1708
   Glu Leu Leu Gly Lys Trp Lys Ser Arg Leu Thr Met Ala Gly Phe Arg
       510                 515                 520
   cct tac ccg ttg agc tcg tac gtc aac tcg gtg ata agg aag ctt ctc    1756
   Pro Tyr Pro Leu Ser Ser Tyr Val Asn Ser Val Ile Arg Lys Leu Leu
   525                 530                 535                 540
   gcc tgc tac tcc gat aaa tac aca ttg gat gag aag gac ggc gcg atg    1804
   Ala Cys Tyr Ser Asp Lys Tyr Thr Leu Asp Glu Lys Asp Gly Ala Met
                   545                 550                 555
   ctt ctc ggc tgg agg agc aga aag ctg ata tct gct tct gcg tgg cac    1852
   Leu Leu Gly Trp Arg Ser Arg Lys Leu Ile Ser Ala Ser Ala Trp His
               560                 565                 570
   tgacagtagt atgttgagaa gaagaaatga tagagagcat atatatacat accgattccc 1912
   tggtccaatt tacagaatga taccctttga tgtaccctca gtttgattga cagcgggtgt 1972
   tgccttcatt tatagaccag gatcaaacat tgacattaga tttgtagaga agttgtatga 2032
   taataatttc tttgcgattt caatgtatga tgtatccatg gctgccatag ttaataatat 2092
   tcttgcatag tcaaaaaaaa aaaaaaaaaa aaaaaaaaaa aaa 2135

<210> 2
<211> 572
<212> PRT
<213> Oryza sativa L cv Nipponbare

<400> 2
Met Asp Leu His Gln Leu Leu Lys Tyr Arg Leu Thr Gly Ala Asn Val
1               5                   10                  15

Val Tyr Glu Ile Pro Thr Glu Asn Asn Leu Gln Asn Ser Pro Trp Gln
            20                  25                  30

Ala Asn Pro Leu Lys Tyr Glu Phe Ser Asp Ser Pro Tyr Thr Pro Leu
        35                  40                  45

Ser Ser Gln Phe Glu Cys Asp Asn Leu Ser Ala Leu Thr Asn Thr Pro
    50                  55                  60

Asp Asn Gln Ser Ser Thr Glu Thr Ile Ser Ala Gln Pro Ile Ser Pro

65                70                75                80

Leu Glu Ala Asp Ser Ser Tyr Arg Gln Ala Gly Ile Leu Leu Gln Glu

                85                90                95

Asn Ile Gln Val Gly Ala Asp Pro Leu Tyr Ala Thr Ser Arg His Asn

            100                105                110

Met Gln His Ala Leu Arg Glu Ile Glu Thr Val Leu Met Ala Pro Asp

        115                120                125

Thr Asp Asp Ala Thr Thr Ser Thr Lys His Glu Phe Glu Glu Ile Lys

    130                135                140

Pro Ala Gln Leu Val Arg Gln Arg Ser Arg Thr Trp Ser His Glu Ser

145                150                155                160

Arg Gln Pro Leu Pro Gly Val Gly Arg Ser Gln Phe Ala Ser Gly Gly

                165                170                175

Tyr Pro Thr Ala Ser Tyr Glu Phe Arg Pro Glu Lys Arg Gln Arg Glu

            180                185                190

Leu Arg Glu Asp Pro Gln Ile Ile Val Lys Gln Leu Leu Thr Arg Cys

        195                200                205

Ala Glu Ala Leu Ser Glu Asp Arg Thr Glu Glu Phe His Lys Leu Val
    210                 215                 220
Gln Glu Ala Arg Gly Val Val Ser Ile Asn Gly Glu Pro Ile Gln Arg
225                 230                 235                 240
Leu Gly Ala Tyr Leu Leu Glu Gly Leu Val Ala Arg His Gly Asn Ser
                245                 250                 255
Gly Thr Asn Ile Tyr Arg Ala Leu Lys Cys Arg Glu Pro Glu Ser Lys
            260                 265                 270
Glu Leu Leu Ser Tyr Met Arg Ile Leu Tyr Asn Ile Cys Pro Tyr Phe
        275                 280                 285
Lys Phe Gly Tyr Met Ala Ala Asn Gly Ala Ile Ala Glu Ala Leu Arg
    290                 295                 300
Thr Glu Asn Asn Ile His Ile Ile Asp Phe Gln Ile Ala Gln Gly Thr
305                 310                 315                 320
Gln Trp Ile Thr Leu Ile Gln Ala Leu Ala Ala Arg Pro Gly Gly Pro
                325                 330                 335
Pro Arg Val Arg Ile Thr Gly Ile Asp Asp Pro Val Ser Glu Tyr Ala
            340                 345                 350

Arg Gly Glu Gly Leu Asp Ile Val Gly Lys Met Leu Lys Ser Met Ser
        355                 360                 365
Glu Glu Phe Lys Ile Pro Leu Glu Phe Thr Pro Leu Ser Val Tyr Ala
    370                 375                 380
Thr Gln Val Thr Lys Glu Met Leu Glu Ile Arg Pro Gly Glu Ala Leu
385                 390                 395                 400
Ser Val Asn Phe Thr Leu Gln Leu His His Thr Pro Asp Glu Ser Val
                405                 410                 415
Asp Val Asn Asn Pro Arg Asp Gly Leu Leu Pro Asp Gly Glu Arg Ala
            420                 425                 430
Val Pro Glu Gly Asp Tyr Phe Gly Arg Ala Gly Val Thr His Gln His
        435                 440                 445
Asn Ala Phe Leu Asp Glu Val Trp Gly Asp His Gly Val Leu Leu Arg
    450                 455                 460
His Val Arg Val Asp Arg Leu Pro Thr Cys Arg Gly Thr Thr Arg Arg
465                 470                 475                 480
Gly Ser Ala Trp Ser Ser Thr Ala Ser Pro Arg His Ile Val Asn Ile

                485                 490                 495

Ile Ala Cys Glu Gly Lys Asp Arg Val Glu Arg His Glu Leu Leu Gly

            500                 505                 510

Lys Trp Lys Ser Arg Leu Thr Met Ala Gly Phe Arg Pro Tyr Pro Leu

        515                 520                 525

Ser Ser Tyr Val Asn Ser Val Ile Arg Lys Leu Leu Ala Cys Tyr Ser

    530                 535                 540

Asp Lys Tyr Thr Leu Asp Glu Lys Asp Gly Ala Met Leu Leu Gly Trp

545                 550                 555                 560

Arg Ser Arg Lys Leu Ile Ser Ala Ser Ala Trp His

                565                 570

<210> 3

<211> 2204

<212> DNA

<213> Oryza sativa L cv Nipponbare

<220>

<221> CDS

<222> (301)..(1932)

<400> 3

cccgcgtccg cccacgcgtc cgcaccacct gcttcctccg cggctgcgtg cgccgcctcc 60

ccaacccttc gtcctccccg ccggccgccg ctgagatccg acgcgccgga gcggagctcg 120

ctgagatttt gttcctgctg gatttctgaa attttactgg aggactagag atatcaagta 180

caccaacaac aaactagtac aaagttttcc ttccagttaa tctgttcctg gatctccttg 240

ccatacgcga agcagtggat actatgcagt aattggttag gaggtcttta ctgaaccata 300

atg gct gat act cca act tcc cga atg att cat ccc ttc agc aat ata 348

Met Ala Asp Thr Pro Thr Ser Arg Met Ile His Pro Phe Ser Asn Ile

1               5                   10                  15

ccg agc caa aac ctg aag cag ttc cag tat tct gat aat cca cag cac 396

Pro Ser Gln Asn Leu Lys Gln Phe Gln Tyr Ser Asp Asn Pro Gln His

            20                  25                  30

cct tgc cac cct tat agg gca ccc tca gac acc cac gtt gtg cca cat 444

Pro Cys His Pro Tyr Arg Ala Pro Ser Asp Thr His Val Val Pro His

        35                  40                  45

cat tat ggc cta aag tca cat tca ccg gat gct ggt tat gaa agc cag 492

His Tyr Gly Leu Lys Ser His Ser Pro Asp Ala Gly Tyr Glu Ser Gln

    50                  55                  60

gct act cct aac aag tac aca ttg gac tcc tct gaa ggt gct ggt tgt 540
Ala Thr Pro Asn Lys Tyr Thr Leu Asp Ser Ser Glu Gly Ala Gly Cys
65                  70                  75                  80

atg aga cat gat tct cct tcc agt caa agt ttc aca acc aga agt ggt 588
Met Arg His Asp Ser Pro Ser Ser Gln Ser Phe Thr Thr Arg Ser Gly
                85                  90                  95

agc cct cta tct cag gaa gac agc cac tct gac tcg act gat gga tct 636
Ser Pro Leu Ser Gln Glu Asp Ser His Ser Asp Ser Thr Asp Gly Ser
            100                 105                 110

cct gta ggc gcc tcc tgt gtc act gag gat cct aat gat ctg aag caa 684
Pro Val Gly Ala Ser Cys Val Thr Glu Asp Pro Asn Asp Leu Lys Gln
        115                 120                 125

aaa ctg aag gat ctc gag gcc gtt atg ctt ggg cca gac tcg gaa ata 732
Lys Leu Lys Asp Leu Glu Ala Val Met Leu Gly Pro Asp Ser Glu Ile
    130                 135                 140

gtc aac agc ctt gag aat tcg gtg gca aac caa ctt tcc ttg gag ccg 780
Val Asn Ser Leu Glu Asn Ser Val Ala Asn Gln Leu Ser Leu Glu Pro
145                 150                 155                 160

gag aag tgg gtg cgc atg atg ggc att cct aga ggc aac ttg aaa gag 828
Glu Lys Trp Val Arg Met Met Gly Ile Pro Arg Gly Asn Leu Lys Glu
                165                 170                 175

cta ctg att gcc tgt gct aga gct gtg gaa gag aag aat agc ttt gct 876
Leu Leu Ile Ala Cys Ala Arg Ala Val Glu Glu Lys Asn Ser Phe Ala
            180                 185                 190

att gat atg atg att cca gag ctg aga aaa ata gtt tcg gta tct ggt 924
Ile Asp Met Met Ile Pro Glu Leu Arg Lys Ile Val Ser Val Ser Gly
        195                 200                 205

gag cca ctt gag agg ttg gga gcc tac atg gta gaa ggg ctt gtt gcc 972
Glu Pro Leu Glu Arg Leu Gly Ala Tyr Met Val Glu Gly Leu Val Ala
    210                 215                 220

agg ctt gcc tcc tct ggc att tca atc tac aaa gct ttg aaa tgc aag 1020
Arg Leu Ala Ser Ser Gly Ile Ser Ile Tyr Lys Ala Leu Lys Cys Lys
225                 230                 235                 240

gaa cca aag agc tct gac ctt ctg tct tac atg cac ttc ctg tat gag 1068
Glu Pro Lys Ser Ser Asp Leu Leu Ser Tyr Met His Phe Leu Tyr Glu
                245                 250                 255

gcc tgt ccc tac ttc aag ttt ggg tat atg tcg gca aat ggt gct att 1116
Ala Cys Pro Tyr Phe Lys Phe Gly Tyr Met Ser Ala Asn Gly Ala Ile

            260                 265                 270

gca gag gct gtc aag gga gaa gac agg att cat atc atc gac ttc cat 1164
Ala Glu Ala Val Lys Gly Glu Asp Arg Ile His Ile Ile Asp Phe His
        275                 280                 285

atc tct caa ggg gct cag tgg atc tct ctc ctt cag gcc ctt gca gcc 1212
Ile Ser Gln Gly Ala Gln Trp Ile Ser Leu Leu Gln Ala Leu Ala Ala
    290                 295                 300

agg cct ggt gga cca cca act gta agg atc act ggt att gat gac tca 1260
Arg Pro Gly Gly Pro Pro Thr Val Arg Ile Thr Gly Ile Asp Asp Ser
305                 310                 315                 320

gtg tca gct tac gca cga ggc ggc ggg cta gag ctg gtc gga agg agg 1308
Val Ser Ala Tyr Ala Arg Gly Gly Gly Leu Glu Leu Val Gly Arg Arg
                325                 330                 335

ctg tcg cac atc gcc agc ctg tgc aag gtg ccc ttt gaa ttc cac cca 1356
Leu Ser His Ile Ala Ser Leu Cys Lys Val Pro Phe Glu Phe His Pro
            340                 345                 350

ctt gct atc tcc ggc agc aag gtg gag gca gca cat ctc gga gtc atc 1404
Leu Ala Ile Ser Gly Ser Lys Val Glu Ala Ala His Leu Gly Val Ile
        355                 360                 365

ccc ggg gaa gcc ctc gcc gtg aac ttc acc ctg gag ctg cac cac att 1452
Pro Gly Glu Ala Leu Ala Val Asn Phe Thr Leu Glu Leu His His Ile
    370                 375                 380

cca gac gag tcg gtg agc acg gcg aac cac cgt gac cgc ctc cta agg 1500
Pro Asp Glu Ser Val Ser Thr Ala Asn His Arg Asp Arg Leu Leu Arg
385                 390                 395                 400

atg gtg aag agc ctg tcg ccg aag gta ctc acc ctg gtg gag atg gag 1548
Met Val Lys Ser Leu Ser Pro Lys Val Leu Thr Leu Val Glu Met Glu
                405                 410                 415

tcc aac acg aac acg gcg cca ttc ccg cag agg ttc gcg gag acg ctg 1596
Ser Asn Thr Asn Thr Ala Pro Phe Pro Gln Arg Phe Ala Glu Thr Leu
            420                 425                 430

gac tac tac acc gcc atc ttc gag tcc atc gac ctg acg ctg ccg agg 1644
Asp Tyr Tyr Thr Ala Ile Phe Glu Ser Ile Asp Leu Thr Leu Pro Arg
        435                 440                 445

gac gac agg gag agg atc aac atg gag cag cac tgc ctc gcg agg gag 1692
Asp Asp Arg Glu Arg Ile Asn Met Glu Gln His Cys Leu Ala Arg Glu
    450                 455                 460

atc gtc aac ctc atc gcg tgc gag ggg gag gag agg gcg gag cgg tac 1740
Ile Val Asn Leu Ile Ala Cys Glu Gly Glu Glu Arg Ala Glu Arg Tyr

465                 470                 475                 480

gag ccc ttc ggc aag tgg aag gcg cgc ctc acc atg gcc ggg ttc agg    1788
Glu Pro Phe Gly Lys Trp Lys Ala Arg Leu Thr Met Ala Gly Phe Arg
                485                 490                 495

ccg tcg ccg ctc agc tcg ctg gtg aac gcc acc atc agg acg ctg ctg    1836
Pro Ser Pro Leu Ser Ser Leu Val Asn Ala Thr Ile Arg Thr Leu Leu
            500                 505                 510

cag agc tac tcg gac aac tac aag ctc gcc gag agg gat ggg gcg ctc    1884
Gln Ser Tyr Ser Asp Asn Tyr Lys Leu Ala Glu Arg Asp Gly Ala Leu
        515                 520                 525

tac ctc ggg tgg aag agc agg ccc ctg gtg gtc tcg tcg gcg tgg cac    1932
Tyr Leu Gly Trp Lys Ser Arg Pro Leu Val Val Ser Ser Ala Trp His
    530                 535                 540

tagcgttgtg actttgtgag gtgatctgac gacgaacttc ccttgtaacc atcttggctg 1992

ggggctttag aaagctatag tgatcatgga ctggtaaggg tagctgtaac aatggtgttg 2052

taatggtaag gtancggcta gggtcaaact cgaaaatggg caaaatgctg atnaggtgta 2112

naagtgtagt acatgtttna nagtatatgt ttcagttttt cttttaaaaa aaaaaaaaaa 2172

aaaaaaaaaa aaaaaaaaaa aaaaaaaaaa aa 2204

<210> 4

<211> 544

<212> PRT

<213> Oryza sativa cv Nipponbare

<400> 4

Met Ala Asp Thr Pro Thr Ser Arg Met Ile His Pro Phe Ser Asn Ile
1               5                   10                  15

Pro Ser Gln Asn Leu Lys Gln Phe Gln Tyr Ser Asp Asn Pro Gln His
            20                  25                  30

Pro Cys His Pro Tyr Arg Ala Pro Ser Asp Thr His Val Val Pro His
        35                  40                  45

His Tyr Gly Leu Lys Ser His Ser Pro Asp Ala Gly Tyr Glu Ser Gln
    50                  55                  60

Ala Thr Pro Asn Lys Tyr Thr Leu Asp Ser Ser Glu Gly Ala Gly Cys
65                  70                  75                  80

Met Arg His Asp Ser Pro Ser Ser Gln Ser Phe Thr Thr Arg Ser Gly
                85                  90                  95

Ser Pro Leu Ser Gln Glu Asp Ser His Ser Asp Ser Thr Asp Gly Ser
            100                 105                 110
Pro Val Gly Ala Ser Cys Val Thr Glu Asp Pro Asn Asp Leu Lys Gln
        115                 120                 125
Lys Leu Lys Asp Leu Glu Ala Val Met Leu Gly Pro Asp Ser Glu Ile
    130                 135                 140
Val Asn Ser Leu Glu Asn Ser Val Ala Asn Gln Leu Ser Leu Glu Pro
145                 150                 155                 160
Glu Lys Trp Val Arg Met Met Gly Ile Pro Arg Gly Asn Leu Lys Glu
                165                 170                 175
Leu Leu Ile Ala Cys Ala Arg Ala Val Glu Glu Lys Asn Ser Phe Ala
            180                 185                 190
Ile Asp Met Met Ile Pro Glu Leu Arg Lys Ile Val Ser Val Ser Gly
        195                 200                 205
Glu Pro Leu Glu Arg Leu Gly Ala Tyr Met Val Glu Gly Leu Val Ala
    210                 215                 220
Arg Leu Ala Ser Ser Gly Ile Ser Ile Tyr Lys Ala Leu Lys Cys Lys

225                 230                 235                 240

Glu Pro Lys Ser Ser Asp Leu Leu Ser Tyr Met His Phe Leu Tyr Glu

                245                 250                 255

Ala Cys Pro Tyr Phe Lys Phe Gly Tyr Met Ser Ala Asn Gly Ala Ile

            260                 265                 270

Ala Glu Ala Val Lys Gly Glu Asp Arg Ile His Ile Ile Asp Phe His

        275                 280                 285

Ile Ser Gln Gly Ala Gln Trp Ile Ser Leu Leu Gln Ala Leu Ala Ala

    290                 295                 300

Arg Pro Gly Gly Pro Pro Thr Val Arg Ile Thr Gly Ile Asp Asp Ser

305                 310                 315                 320

Val Ser Ala Tyr Ala Arg Gly Gly Gly Leu Glu Leu Val Gly Arg Arg

                325                 330                 335

Leu Ser His Ile Ala Ser Leu Cys Lys Val Pro Phe Glu Phe His Pro

            340                 345                 350

Leu Ala Ile Ser Gly Ser Lys Val Glu Ala Ala His Leu Gly Val Ile

        355                 360                 365

Pro Gly Glu Ala Leu Ala Val Asn Phe Thr Leu Glu Leu His His Ile
    370                 375                 380

Pro Asp Glu Ser Val Ser Thr Ala Asn His Arg Asp Arg Leu Leu Arg
385                 390                 395                 400

Met Val Lys Ser Leu Ser Pro Lys Val Leu Thr Leu Val Glu Met Glu
                405                 410                 415

Ser Asn Thr Asn Thr Ala Pro Phe Pro Gln Arg Phe Ala Glu Thr Leu
            420                 425                 430

Asp Tyr Tyr Thr Ala Ile Phe Glu Ser Ile Asp Leu Thr Leu Pro Arg
        435                 440                 445

Asp Asp Arg Glu Arg Ile Asn Met Glu Gln His Cys Leu Ala Arg Glu
    450                 455                 460

Ile Val Asn Leu Ile Ala Cys Glu Gly Glu Glu Arg Ala Glu Arg Tyr
465                 470                 475                 480

Glu Pro Phe Gly Lys Trp Lys Ala Arg Leu Thr Met Ala Gly Phe Arg
                485                 490                 495

Pro Ser Pro Leu Ser Ser Leu Val Asn Ala Thr Ile Arg Thr Leu Leu
            500                 505                 510

Gln Ser Tyr Ser Asp Asn Tyr Lys Leu Ala Glu Arg Asp Gly Ala Leu
        515                 520                 525

Tyr Leu Gly Trp Lys Ser Arg Pro Leu Val Val Ser Ser Ala Trp His
    530                 535                 540

<210> 5
<211> 625
<212> PRT
<213> Oryza sativa L cv Nipponbare

<400> 5
Met Lys Arg Glu Tyr Gln Glu Ala Gly Gly Ser Ser Gly Gly Gly Ser
1               5                   10                  15

Ser Ala Asp Met Gly Ser Cys Lys Asp Lys Val Met Ala Gly Ala Ala
            20                  25                  30

Gly Glu Glu Glu Asp Val Asp Glu Leu Leu Ala Ala Leu Gly Tyr Lys
        35                  40                  45

Val Arg Ser Ser Asp Met Ala Asp Val Ala Gln Lys Leu Glu Gln Leu
    50                  55                  60

Glu Met Ala Met Gly Met Gly Gly Val Ser Ala Pro Gly Ala Ala Asp
65                  70                  75                  80

Asp Gly Phe Val Ser His Leu Ala Thr Asp Thr Val His Tyr Asn Pro
                85                  90                  95

Ser Asp Leu Ser Ser Trp Val Glu Ser Met Leu Ser Glu Leu Asn Ala
            100                 105                 110

Pro Leu Pro Pro Ile Pro Pro Ala Pro Pro Ala Ala Arg His Ala Ser
        115                 120                 125

Thr Ser Ser Thr Val Thr Gly Gly Gly Gly Ser Gly Phe Phe Glu Leu
    130                 135                 140

Pro Ala Ala Ala Asp Ser Ser Ser Ser Thr Tyr Ala Leu Arg Pro Ile
145                 150                 155                 160

Ser Leu Pro Val Val Ala Thr Ala Asp Pro Ser Ala Ala Asp Ser Ala
                165                 170                 175

Arg Asp Thr Lys Arg Met Arg Thr Gly Gly Gly Ser Thr Ser Ser Ser
            180                 185                 190

Ser Ser Ser Ser Ser Ser Leu Gly Gly Gly Ala Ser Arg Gly Ser Val
        195                 200                 205

Val Glu Ala Ala Pro Pro Ala Thr Gln Gly Ala Ala Ala Ala Asn Ala
    210                 215                 220
Pro Ala Val Pro Val Val Val Val Asp Thr Gln Glu Ala Gly Ile Arg
225                 230                 235                 240
Leu Val His Ala Leu Leu Ala Cys Ala Glu Ala Val Gln Gln Glu Asn
                245                 250                 255
Phe Ala Ala Ala Glu Ala Leu Val Lys Gln Ile Pro Thr Leu Ala Ala
            260                 265                 270
Ser Gln Gly Gly Ala Met Arg Lys Val Ala Ala Tyr Phe Gly Glu Ala
        275                 280                 285
Leu Ala Arg Arg Val Tyr Arg Phe Arg Pro Ala Asp Ser Thr Leu Leu
    290                 295                 300
Asp Ala Ala Phe Ala Asp Leu Leu His Ala His Phe Tyr Glu Ser Cys
305                 310                 315                 320
Pro Tyr Leu Lys Phe Ala His Phe Thr Ala Asn Gln Ala Ile Leu Glu
                325                 330                 335
Ala Phe Ala Gly Cys His Arg Val His Val Val Asp Phe Gly Ile Lys

            340                 345                 350

Gln Gly Met Gln Trp Pro Ala Leu Leu Gln Ala Leu Ala Leu Arg Pro

        355                 360                 365

Gly Gly Pro Pro Ser Phe Arg Leu Thr Gly Val Gly Pro Pro Gln Pro

    370                 375                 380

Asp Glu Thr Asp Ala Leu Gln Gln Val Gly Trp Lys Leu Ala Gln Phe

385                 390                 395                 400

Ala His Thr Ile Arg Val Asp Phe Gln Tyr Arg Gly Leu Val Ala Ala

                405                 410                 415

Thr Leu Ala Asp Leu Glu Pro Phe Met Leu Gln Pro Glu Gly Glu Ala

            420                 425                 430

Asp Ala Asn Glu Glu Pro Glu Val Ile Ala Val Asn Ser Val Phe Glu

        435                 440                 445

Leu His Arg Leu Leu Ala Gln Pro Gly Ala Leu Glu Lys Val Leu Gly

    450                 455                 460

Thr Val His Ala Val Arg Pro Arg Ile Val Thr Val Val Glu Gln Glu

465                 470                 475                 480

Ala Asn His Asn Ser Gly Ser Phe Leu Asp Arg Phe Thr Glu Ser Leu
                485                 490                 495
His Tyr Tyr Ser Thr Met Phe Asp Ser Leu Glu Gly Gly Ser Ser Gly
            500                 505                 510
Gln Ala Glu Leu Ser Pro Pro Ala Ala Gly Gly Gly Gly Gly Thr Asp
        515                 520                 525
Gln Val Met Ser Glu Val Tyr Leu Gly Arg Gln Ile Cys Asn Val Val
    530                 535                 540
Ala Cys Glu Gly Ala Glu Arg Thr Glu Arg His Glu Thr Leu Gly Gln
545                 550                 555                 560
Trp Arg Asn Arg Leu Gly Arg Ala Gly Phe Glu Pro Val His Leu Gly
                565                 570                 575
Ser Asn Ala Tyr Lys Gln Ala Ser Thr Leu Leu Ala Leu Phe Ala Gly
            580                 585                 590
Gly Asp Gly Tyr Arg Val Glu Glu Lys Glu Gly Cys Leu Thr Leu Gly
        595                 600                 605
Trp His Thr Arg Pro Leu Ile Ala Thr Ser Ala Trp Arg Val Ala Ala
    610                 615                 620

Ala

625

<210> 6

<211> 427

<212> PRT

<213> Lycopersicon esculentum

<400> 6

Leu Gly Ser Phe Gly Ser Ser Ser Ser Gln Ser His Pro His His Asp

1               5                   10                  15

Glu Glu Ser Ser Asp His His Gln Gln Arg Arg Phe Thr Ala Thr Ala

            20                  25                  30

Thr Thr Ile Thr Thr Thr Thr Ile Thr Thr Ser Pro Ala Ile Gln Ile

        35                  40                  45

Arg Gln Leu Leu Ile Ser Cys Ala Glu Leu Ile Ser Gln Ser Asp Phe

    50                  55                  60

Ser Ala Ala Lys Arg Leu Leu Thr Ile Leu Ser Thr Asn Ser Ser Pro

65                  70                  75                  80

Phe Gly Asp Ser Thr Glu Arg Leu Val His Gln Phe Thr Arg Ala Leu
                85                  90                  95
Ser Leu Arg Leu Asn Arg Tyr Ile Ser Ser Thr Thr Asn His Phe Met
            100                 105                 110
Thr Pro Val Glu Thr Thr Pro Thr Asp Ser Ser Ser Ser Ser Ser Leu
        115                 120                 125
Ala Leu Ile Gln Ser Ser Tyr Leu Ser Leu Asn Gln Val Thr Pro Phe
    130                 135                 140
Ile Arg Phe Thr Gln Leu Thr Ala Asn Gln Ala Ile Leu Glu Ala Ile
145                 150                 155                 160
Asn Gly Asn His Gln Ala Ile His Ile Val Asp Phe Asp Ile Asn His
                165                 170                 175
Gly Val Gln Trp Pro Pro Leu Met Gln Ala Leu Ala Asp Arg Tyr Pro
            180                 185                 190
Ala Pro Thr Leu Arg Ile Thr Gly Thr Gly Asn Asp Leu Asp Thr Leu
        195                 200                 205
Arg Arg Thr Gly Asp Arg Leu Ala Lys Phe Ala His Ser Leu Gly Leu

    210                 215                 220

Arg Phe Gln Phe His Pro Leu Tyr Ile Ala Asn Asn Asn His Asp His
225                 230                 235                 240

Asp Glu Asp Pro Ser Ile Ile Ser Ser Ile Val Leu Leu Pro Asp Glu
                245                 250                 255

Thr Leu Ala Ile Asn Cys Val Phe Tyr Leu His Arg Leu Leu Lys Asp
            260                 265                 270

Arg Glu Lys Leu Arg Ile Phe Leu His Arg Val Lys Ser Met Asn Pro
        275                 280                 285

Lys Ile Val Thr Ile Ala Glu Lys Glu Ala Asn His Asn His Pro Leu
    290                 295                 300

Phe Leu Gln Arg Phe Ile Glu Ala Leu Asp Tyr Tyr Thr Ala Val Phe
305                 310                 315                 320

Asp Ser Leu Glu Ala Thr Leu Pro Pro Gly Ser Arg Glu Arg Met Thr
                325                 330                 335

Val Glu Gln Val Trp Phe Gly Arg Glu Ile Val Asp Ile Val Ala Met
            340                 345                 350

Glu Gly Asp Lys Arg Lys Glu Arg His Glu Arg Phe Arg Ser Trp Glu

        355                 360                 365

Val Met Leu Arg Ser Cys Gly Phe Ser Asn Val Ala Leu Ser Pro Phe

    370                 375                 380

Ala Leu Ser Gln Ala Lys Leu Leu Leu Arg Leu His Tyr Pro Ser Glu

385                 390                 395                 400

Gly Tyr Gln Leu Gly Val Ser Ser Asn Ser Phe Phe Leu Gly Trp Gln

                405                 410                 415

Asn Gln Pro Leu Phe Ser Ile Ser Ser Trp Arg

            420                 425